# EUROPÄISCHE PATENTSCHRIFT

(11) **EP 0 935 454 B1**
(45) Veröffentlichungstag und Bekanntmachung des Hinweises auf die Patenterteilung: **22.08.2001**
(21) Anmeldenummer: 97927111.1
(22) Anmeldetag: 03.06.1997
(51) Int. Cl.: A61K 7/48

(54) **KOSMETISCHE UND/ODER PHARMAZEUTISCHE ZUBEREITUNGEN**
COSMETIC AND/OR PHARMACEUTICAL PREPARATIONS
PREPARATIONS COSMETIQUES ET/OU PHARMACEUTIQUES

(30) Priorität: 12.06.1996 DE 19623383; 12.03.1997 DE 19710154
(43) Veröffentlichungstag der Anmeldung: 18.08.1999
(73) Patentinhaber: Cognis Deutschland GmbH, 40589 Düsseldorf (DE)
(72) Erfinder: LÖHL, Thorsten, D-40597 Düsseldorf (DE); GASSENMEIER, Thomas, D-40229 Düsseldorf (DE); KAHRE, Jörg, D-42799 Leichlingen (DE); HENSEN, Hermann, D-42781 Haan (DE); TESMANN, Holger, D-41363 Jüchen (DE); ANSMANN, Achim, D-40699 Erkrath (DE)
(86) Internationale Anmeldenummer: EP9702867
(87) Internationale Veröffentlichungsnummer: WO9747282

(56) Entgegenhaltungen:
- DE-A- 4 040 154
- DE-A- 4 119 890
- DE-A- 4 317 683
- US-A- 4 395 370
- DATABASE WPI Week 9601 Derwent Publications Ltd., London, GB; AN 96-006868 XP002042612 "Oily cosmetic, used as lip-stick, foundation cream, eye shadow etc.- contg. dialkyl carbonate(s) and polyether denatured silicone" & JP 07 285 829 A (KANEBO) , 31.Oktober 1995

## Beschreibung

Die Erfindung betrifft kosmetische bzw. pharmazeutische Zubereitungen, die als Ölkörper ausgewählte Dialkylcarbonate sowie weiterhin Emulgatoren, vorzugsweise Alkyloligoglucoside enthalten und weiterhin die Verwendung der Dialkylcarbonate als Ersatzstoffe für Siliconöle.

### Stand der Technik

Silicone werden in der Haut- und Haarkosmetik als Additive zur Beeinflussung von Griff und Glanz eingesetzt. Nachteilig hierbei ist jedoch der sogenannte "build-up"-Effekt, Hierunter ist zu verstehen, daß bei wiederholter Anwendung siliconhaltiger Produkte sich auf der Haut oder dem Haar eine Schicht von Polymeren aufbaut, welche durch einfaches Waschen nur schwer zu entfernen ist. Insbesondere beim Haar kann es dabei zu einer Belastung und einer möglichen Beeinträchtigung weiterer Behandlungen wie z.B. beim Wellen oder Färben kommen. Ebenfalls von Nachteil ist die unzulängliche biologische Abbaubarkeit vieler Silicontypen. Eine Übersicht zum Einsatz von Siliconen in der Kosmetik findet sich beispielsweise von K.Schnurrbusch in **Seifen-Fette-Öle-Wachse 100, 173, 1974).**

Aus der deutschen Offenlegungsschrift **DE-A1 4040154** (Henkel) ist der Einsatz von Guerbetcarbonaten auf Basis von Alkoholen mit mindestens 6 Kohlenstoffatomen als Ölkörper für kosmetische Mittel bekannt. Gegenstand der deutschen Offenlegungsschrift **DE-A1 4317683** (Henkel) sind Carbonate auf Basis von ethoxylierten Fettalkoholen und ihr Einsatz zur Reinigung harter Oberflächen.

Die Aufgabe der Erfindung hat somit darin bestanden, Ersatzstoffe für Silicone zu finden, die sich bei der Anwendung nicht anreichern und dennoch hinsichtlich Griff und Glanz mindestens vergleichbare anwendungstechnische Eigenschaften aufweisen.

### Beschreibung der Erfindung

Gegenstand der Erfindung sind kosmetische und/oder pharmazeutische Zubereitungen, enthaltend
(a) Dialkylcarbonate der Formel (**I**), 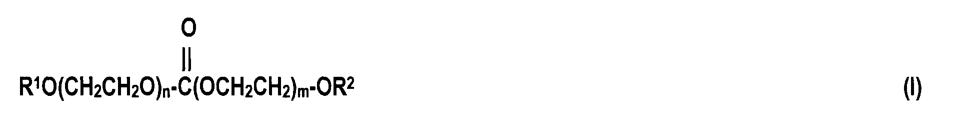 in der R¹ für einen linearen Alkyl- und/oder Alkenylrest mit 6 bis 22 Kohlenstoffatomen oder einen Rest, der sich von einem Polyol mit 2 bis 15 Kohlenstoffatomen und mindestens zwei Hydroxylgruppen ableitet, R² für R¹ oder Methyl steht und n und m unabhängig voneinander 0 oder Zahlen von 1 bis 100 bedeuten, und
(b) Emulgatoren.

Überraschenderweise wurde gefunden, daß die Zubereitungen mit den ausgewählten Dialkylcarbonaten als Ölkörpern im Vergleich zu Siliconölen hinsichtlich Griff und Glanz mindestens vergleichbar gut sensorisch beurteilt werden, ohne daß es auf Haut und Haaren zu einer unerwünschten Anreicherung kommt. Die Erfindung schließt ferner die Erkenntnis ein, daß die Dialkylcarbonate der Formel (**I**) ein breites Spektrum unterschiedlicher sensorischer Eigenschaften abdecken können.

### Dialkylcarbonate

Dialkylcarbonate stellen grundsätzlich bekannte Stoffe dar, auch wenn einige der beanspruchten Carbonate an dieser Stelle erstmals beschrieben werden. Grundsätzlich lassen sich die Stoffe durch Umesterung von Dimethyl- oder Diethylcarbonat mit den genannten Hydroxyverbindungen nach den Verfahren des Stands der Technik herstellen; eine Übersicht hierzu findet sich beispielsweise in **Chem.Rev. 96, 951 (1996).** Dialkylcarbonate der Formel (**I**), die sich in besonderer Weise zur Lösung der vorliegenden Aufgabenstellung eignen, erfüllen eine der folgenden Bedingungen:
(a) R¹ steht für einen linearen Alkylrest mit 8 bis 18 Kohlenstoffatomen und R² für R¹ oder Methyl;
(b) R¹ steht für einen linearen Alkylrest mit 12 bis 18 Kohlenstoffatomen, R² für R¹ oder Methyl und n und m jeweils für Zahlen von 1 bis 10;
(c) R¹ steht für einen Rest eine Polyols steht, welches ausgewählt ist aus der Gruppe, die gebildet wird von Glycerin, Alkylenglycolen, technischen Oligoglyceringemischen, Methylolverbindungen, Niedrigalkylglucosiden, Zuckeralkoholen, Zuckern und Aminozuckern, und R² für R¹, einen linearen Alkylrest mit 8 bis 12 Kohlenstoffatomen oder Methyl.

Typische Beispiele für Dialkylcarbonate der beiden Gruppen (a) und (b) sind vollständige oder partielle Umesterungsprodukte von Dimethyl- und/oder Diethylcarbonat mit Capronalkohol, Caprylalkohol, 2-Ethylhexylalkohol, Caprinalkohol, Laurylalkohol, Isotridecylalkohol, Myristylalkohol, Cetylalkohol, Palm-oleylalkohol, Stearylalkohol, Isostearylalkohol, Oleylalkohol, Elaidylalkohol, Petroselinylalkohol, Linolyl-alkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen, In gleicher Weise geeignet sind die Umesterungsprodukte der niederen Carbonate mit den genannten Alkoholen in Form ihrer Addukte mit 1 bis 100, vorzugsweise 2 bis 50 und insbesondere 5 bis 20 Mol Ethylenoxid.

Die Carbonate der Gruppe (c) werden hier erstmals beschrieben. Es handelt sich um Stoffe, die erhalten werden indem man Dimethyl- und/oder Diethylcarbonat ganz oder partiell mit Polyolen umestert. Polyole, die im Sinne der Erfindung in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Bei dieser Reaktion kann es natürlich nicht nur zum Austausch einer Methyl- oder Ethylgruppe gegen einen Polyolrest kommen, vielmehr wird ein Gemisch erhalten, bei dem mehrere oder sogar alle Hydroxylgruppen des Polyols mit Carbonatgruppen verknüpft sind, so daß sich gegebenenfalls sogar eine oligomere bzw. polymere Netzstruktur ergibt. Stoffe dieser Art sollen im Sinne der Erfindung ebenfalls durch die allgemeine Formel (**I**) umfaßt werden.

### Emulgatoren

Als Emulgatoren kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin,
(13) Polyalkylenglycole sowie
(14) hydrophobierte Polyacrylate z.B. vom Pemulen-Typ.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 2024051** als Rückfettungsmittel für kosmetische Zubereitungen bekannt.

**Alkyl- und Alkenyloligoglykoside** stellen bekannte nichtionische Tenside dar, die der Formel (**II**) folgen,

**R**^{**3**}**O-[G]**_{**p**} (II)

in der R³ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie, beispielsweise durch sauer katalysierte Acetalisierung von Glucose mit Fettalkoholen erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo-**glucoside.** Die Indexzahl p in der allgemeinen Formel (**II**) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R³ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R³ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Weiterhin können als Emulgatoren zwitterionische Tenside von Typ der **Betaine** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-amino-propyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH-oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyl-iminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind. In Summe bevorzugt ist der Einsatz von Alkyloligoglucosiden, Betainen und Polyglycerinpoly-12-hydroxystearaten.

Üblicherweise enthalten die Zubereitungen die Dialkylcarbonate und die Emulgatoren jeweils in Mengen von 0,1 bis 20, vorzugsweise 1 bis 10 Gew.-% - bezogen auf die Zubereitungen, wobei das Gewichtsverhältnis zwischen den Dialkylcarbonaten und den Emulgatoren in der Regel bei 30 : 1 bis 1 : 20, vorzugsweise 5 : 1 bis 1 : 10 liegt. Bei rinse-off-Produkten beträgt das Einsatzverhältnis vorzugsweise 1 : 5 bis 1 : 10, bei leave-on-Produkten 5 : 1 bis 1:1.

### Gewerbliche Anwendbarkeit

Die im Sinne der Erfindung einzusetzenden Dialkylcarbonate besitzen ausgezeichnete sensorische Eigenschaften auf Haut und Haaren, die denen von Siliconölen in nichts nachstehen. Ein weiterer Gegenstand der Erfindung betrifft daher ihre Verwendung als Ersatzstoffe für Siliconöle zur Herstellung von kosmetischen und/oder pharmazeutischen Zubereitungen.

### Kosmetische und/oder pharmazeutische Zubereitungen

Die erfindungsgemäßen Zubereitungen, wie beispielsweise Haarshampoos, Haarlotionen, Schaumbäder, Cremes, Lotionen oder Salben, können ferner als weitere Hilfs- und Zusatzstoffe milde Tenside, weitere Ölkörper, Überfettungsmittel, Stabilisatoren, Wachse, Konsistenzgeber, Verdickungsmittel, Kationpolymere, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Farb- und Duftstoffe enthalten.

Typische Beispiele für geeignete milde, d.h. besonders hautverträgliche **Tenside** sind Fettalkoholpoly-glycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als weitere **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate Guerbetcarbonate, Dialkylether, Siliconöle und/oder aliphatische bzw. naphthenische Kohlenwasserstoffe in Betracht.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentrimamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol,

Nachdem die Aufgabe der Erfindung darin u.a. bestanden hat, **Siliconverbindungen** in den Zubereitungen zu ersetzen, ist die zusätzliche Anwesenheit dieser Stoffe zwar nicht erforderlich, aber nicht grundsätzlich ausgeschlossen. Geeignete Siliconverbindungen sind beispielsweise Dimethyl-polysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Pflanzenextrakte und Vitamin-komplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Unter **UV-Lichtschutzfiltern** sind organische Substanzen zu verstehen, die in der Lage sind, ultraviolette Strahlen zu absorbieren und die aufgenommene Energie in Form iängerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxy-zimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Verschiedene Haarshampoos mit Dialkylcarbonaten (erfindungsgemäße Rezepturen R1 bis R7) bzw. Silicon (Vergleichsrezeptur R8) wurden von einem Panel von 20 Probanden im bekannten Halbseitentest bezüglich Griff und Glanz auf einer Skala von 1 (= angenehmer Weichgriff, brillanter Glanz) bis 5 (= hart, stumpf) beurteilt. Zur qualitativen Bestimmung der Anreicherung der Ölkörper auf dem Haar wurden Haarsträhnen 10mal abwechselnd mit den Testrezepturen behandelt und getrocknet und anschließend verascht. Eine starke Anreicherung von Ölkörpern wurde in Tabelle mit (+) gekennzeichnet, waren nur geringe oder gar keine Anteile an Ölkörpern nachweisbar, ist dies mit (-) gekennzeichnet. Die Ergebnisse stellen Mittelwerte dar.

**Tabelle 1**

| **Haarshampoos : Griff und Glanz (Mengenangaben als Gew.-%)** | | | | | | | | |
|---|---|---|---|---|---|---|---|---|
| **Komponenten** | **R1** | **R2** | **R3** | **R4** | **R5** | **R6** | **R7** | **R8** |
| Sodium Laureth Sulfate | 12 | | | | | | | |
| Coco Glucosides | 4 | | | | | | | |
| Cocoamidopropyl Betaine | 2 | | | | | | | |
| PEG Distearate | 3 | | | | | | | |
| Methyloctylcarbonat | 1 | - | - | - | - | - | - | - |
| Di-n-octylcarbonat | - | 1 | - | - | - | - | - | - |
| 2-Ethylhexyl-lauryl+7EOcarbonat | - | - | 1 | - | - | - | - | - |
| Octyllauryl+7EO-carbonat | - | - | - | 1 | - | - | - | - |
| Di-n-lauryl+7EO-carbonat | - | - | - | - | 1 | - | - | - |
| Di-n-cetearyl+5EO-carbonat | - | - | - | - | - | 1 | - | - |
| Tris-(2-ethylhexyl)-trimethylolcarbonat | - | - | - | - | - | - | 1 | - |
| Dimethicone | - | - | - | - | - | - | - | 1 |
| NaCI | 0,5 | | | | | | | |
| Wasser | ad 100 | | | | | | | |
| ***Beurteilung Halbseitentest*** | | | | | | | | |
| - Griff | 2 | 2 | 2 | 1,5 | 2 | 1 | 1 | 2,5 |
| - Glanz | 1 | 2 | 1,5 | 2,5 | 2 | 1 | 1 | 3 |
| - Anreicherung | - | - | - | - | - | - | - | + |

In gleicher Weise wurden die erfindungsgemäßen Haarnachbehandlungsmittel R9 bis R15 sowie das Vergleichsprodukt R16 getestet. Die Ergebnisse sind in Tabelle 2 zusammengefaßt,

**Tabelle 2**

| **Haarnachbehandlungsmittel : Griff und Glanz (Mengenangaben als Gew.-%)** | | | | | | | | |
|---|---|---|---|---|---|---|---|---|
| **Komponenten** | **R9** | **R10** | **R11** | **R12** | **R13** | **R14** | **R15** | **R16** |
| Cetearylalkohol | 3 | | | | | | | |
| Cetrimmonium chloride | 4 | | | | | | | |
| Glyceryl Stearate | 3 | | | | | | | |
| Methyloctylcarbonat | 1 | - | - | - | - | - | - | - |
| Di-n-octylcarbonat | - | 1 | - | - | - | - | - | - |
| 2-Ethylhexyl-lauryl+7EO-carbonat | - | - | 1 | - | - | - | - | - |
| Octyllauryl+7EO-carbonat | - | - | - | 1 | - | - | - | - |
| Di-n-lauryl+7EO-carbonat | - | - | - | - | 1 | - | - | - |
| Di-n-cetearyl+5EO-carbonat | - | - | - | - | - | 1 | - | - |
| Tris-(2-ethylhexyl)-trimethylol-carbonat | - | - | - | - | - | - | 1 | - |
| Dimethicone | - | - | - | - | - | - | - | 1 |
| NaCI | 0,5 | | | | | | | |
| Wasser | ad 100 | | | | | | | |
| ***Beurteilung Halbseitentest*** | | | | | | | | |
| - Griff | 2 | 2 | 1,5 | 1,5 | 2 | 1 | 2,5 | 3 |
| - Glanz | 1 | 1,5 | 1 | 2 | 2 | 1 | 2,5 | 3,5 |
| -Anreicherung | - | - | - | - | - | - | - | + |

Die Paneltests zeigen, daß unter Verwendung der erfindungsgemäßen Fettstoffe Zubereitungen erhalten werden, die in der Anwendung auf dem Haar besser als die Vergleichsrezepturen mit Siliconen beurteilt werden und gleichzeitig den Vorteil aufweisen, daß es nicht zu einem "build up-Effekt" kommt.

Die sensorischen Eigenschaften von O/W-Cremes mit Dialkylcarbonaten bzw. Siliconölen wurden im sogenannten Sensory Assessment untersucht. Die Zusammensetzungen der Zubereitungen sind Tabelle 3 zu entnehmen; die Rezepturen 17 und 20 sind erfindungsgemäß, die Formulierungen 18, 19, 21 und 22 dienen zum Vergleich.

**Tabelle 3**

| **O/W-Cremes (Mengenangaben als Gew.-%)** | | | | | | |
|---|---|---|---|---|---|---|
| **INCI-Bezeichnung** | **R17** | **R18** | **R19** | **R20** | **R21** | **R22** |
| Glyceryl Stearate | 2,5 | 2,5 | 2,5 | 2,7 | 2,7 | 2,7 |
| Cetearyl Alcohol | 3,8 | 3,8 | 3,8 | 4,0 | 4,0 | 4,0 |
| Capric/Caprylic Triglycerides | 3,6 | 3,6 | 3,6 | - | - | - |
| Petrolatum | 12,1 | 12,1 | 12,1 | - | - | - |
| Dioctylcarbonat | 5,0 | - | - | 3,3 | - | - |
| Cyclomethicone | - | 5,0 | - | - | - | - |
| Mineralöl | - | - | 5,0 | 18,7 | 18,7 | 18,7 |
| PEG-20 Glyceryl Stearate | 4,4 | 4,4 | 4,4 | 4,7 | 4,7 | 4,7 |
| Dimethicon | 0,3 | 0,3 | 0,3 | - | 3,3 | - |
| Propylenglycol | 6,3 | 6,3 | 6,3 | - | - | - |
| Wasser | ad 100 | | | | | |

Im Vergleich zu den Vergleichsrezepturen 18 und 19 zeichnet sich die erfindungsgemäße Rezeptur 17 durch ein rascheres Einziehen in die Haut und weniger ölige oder wachsartige Rückstände aus. Im Vergleich zu den Vergleichsrezepturen 21 und 22 werden bei der erfindungsgemäßen Rezeptur 20 geringere Weißeleffekte beobachtet.

## Patentansprüche

1. Kosmetische und/oder pharmazeutische Zubereitungen, enthaltend
(a) Dialkylcarbonate der Formel (**I**), 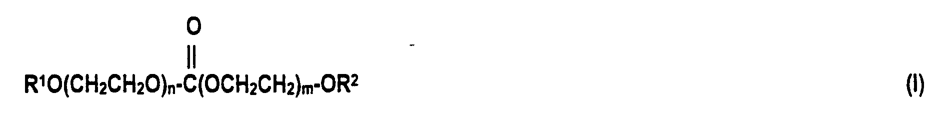 in der R¹ für einen linearen Alkyl- und/oder Alkenylrest mit 6 bis 22 Kohlenstoffatomen oder einen Rest, der sich von einem Polyol mit 2 bis 15 Kohlenstoffatome und mindestens zwei Hydroxylgruppen ableitet, R² für R¹ oder Methyl steht und n und m unabhängig voneinander 0 oder Zahlen von 1 bis 100 bedeuten, und
(b) Emulgatoren.

2. Zubereitungen nach Anspruch 1, **dadurch gekennzeichnet,** daß sie Dialkylcarbonate der Formel (**I**) enthalten, in der R¹ für einen linearen Alkylrest mit 8 bis 18 Kohlenstoffatomen und R² für R¹ oder Methyl steht.

3. Zubereitungen nach Anspruch 1, **dadurch gekennzeichnet,** daß sie Dialkylcarbonate der Formel (**I**) enthalten, in der R¹ für einen linearen Alkylrest mit 12 bis 18 Kohlenstoffatomen, R² für R¹ oder Methyl und n und m jeweils für Zahlen von 1 bis 10 stehen.

4. Zubereitungen nach Anspruch 1, **dadurch gekennzeichnet,** daß sie Dialkylcarbonate der Formel (**I**) enthalten, in der R¹ für einen Rest eine Polyols steht, welches ausgewählt ist aus der Gruppe, die gebildet wird von Glycerin, Alkylenglycolen, technischen Oligoglyceringemischen, Methylolverbindungen, Niedrigalkylglucosiden, Zuckeralkoholen, Zuckern und Aminozuckern, und R² für R¹, einen linearen oder verzweigten Alkylrest mit 8 bis 12 Kohlenstoffatomen oder Methyl steht.

5. Zubereitungen nach den Ansprüchen 1 bis 4, **dadurch gekennzeichnet,** daß sie Emulgatoren enthalten, die ausgewählt sind aus der Gruppe, die gebildet wird von Anlagerungsprodukten von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; C_{12/18}-Fettsäuremono- und -diestern von Anlagerungsprodukten von 1 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und deren Ethylenoxidanlagerungsprodukte; Alkylmono- und -oligoglycosiden mit 8 bis 22 Kohlenstoffatomen im Alkylrest und deren ethoxylierte Analoga; Anlagerungsprodukten von 15 bis 60 Mol Ethylenoxid an Ricinusöl und/oder gehärtetes Ricinusöl; Polyolestern; Anlagerungsprodukten von 2 bis 15 Mol Ethylenoxid an Ricinusöl und/oder gehärtetes Ricinusöl; Partialestern auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkoholen, Alkylglucosiden sowie Polyglucosiden; Trialkylphosphaten sowie Mono-, Di- und/oder Tri-PEG-alkylphosphaten; Wollwachsalkoholen; Polysiloxan-Polyalkyl-Polyether-Copolymeren; Mischestern aus Pentaerythrit, Fettsäuren, Citronensäure und Fettalkohol und/oder Mischestern von Fettsäuren mit 6 bis 22 Kohlenstoffatomen, Methylglucose und Polyolen; hydrophobierten Polyacrylaten, Polyalkylenglycolen; Betainen und Esterquats.

6. Zubereitungen nach Anspruch 5, **dadurch gekennzeichnet,** daß sie als Emulgatoren Alkyloligoglucoside, Betaine und/oder Polyglycerinpoly-12-hydroxystearate enthalten.

7. Zubereitungen nach den Ansprüchen 1 bis 6, **dadurch gekennzeichnet,** daß sie die Dialkylcarbonate und die Emulgatoren jeweils in Mengen von 0,1 bis 20 Gew.-% - bezogen auf die Zubereitungen - enthalten.

8. Zubereitungen nach den Ansprüchen 1 bis 7, **dadurch gekennzeichnet,** daß sie die Dialkylcarbonate und die Emulgatoren im Gewichtsverhältnis 1 : 1 bis 1 : 20 enthalten.

9. Zubereitungen nach den Ansprüchen 1 bis 8, **dadurch gekennzeichnet,** daß sie weiterhin Tenside, Ölkörper, Überfettungsmittel, Stabilisatoren, Wachse, Konsistenzgeber, Verdickungsmittel, Kationpolymere, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Farb- und Duftstoffe enthalten.

10. Verwendung von Dialkylcarbonaten nach Anspruch 1 als Siliconölersatz zur Herstellung von kosmetischen und/oder pharmazeutischen Zubereitungen.

## Claims

1. Cosmetic and/or pharmaceutical compositions containing
(a) dialkyl carbonates corresponding to formula (I): 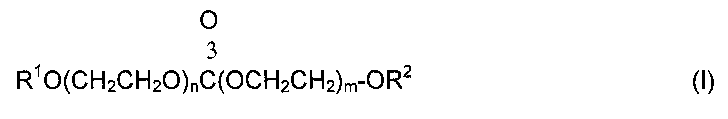 in which R¹ is a linear alkyl and/or alkenyl group containing 6 to 22 carbon atoms and/or a group derived from a polyol containing 2 to 15 carbon atoms and at least two hydroxyl groups, R² has the same meaning as R¹ or is a methyl group and n and m independently of one another stand for 0 or numbers of 1 to 100, and
(b) emulsifiers.

2. Compositions as claimed in claim 1, characterized in that they contain dialkyl carbonates corresponding to formula (I) in which R¹ is a linear alkyl group containing 8 to 18 carbon atoms and R² has the same meaning as R¹ or represents methyl.

3. Compositions as claimed in claim 1, characterized in that they contain dialkyl carbonates corresponding to formula (I) in which R¹ is a linear alkyl group containing 12 to 18 carbon atoms, R² has the same meaning as R¹ or represents methyl and n and m are each numbers of 1 to 10.

4. Compositions as claimed in claim 1, characterized in that they contain dialkyl carbonates corresponding to formula (I) in which R¹ is a residue of a polyol selected from the group consisting of glycerol, alkylene glycols, technical oligoglycerol mixtures, methylol compounds, lower alkyl glucosides, sugar alcohols, sugars and aminosugars and R² has the same meaning as R¹ or represents a linear or branched alkyl group containing 8 to 12 carbon atoms or methyl.

5. Compositions as claimed in claims 1 to 4, characterized in that they contain emulsifiers selected from the group consisting of products of the addition of 2 to 30 moles of ethylene oxide and/or 0 to 5 moles of propylene oxide to linear fatty alcohols containing 8 to 22 carbon atoms, to fatty acids containing 12 to 22 carbon atoms and to alkylphenols containing 8 to 15 carbon atoms in the alkyl group; C_{12/18} fatty acid monoesters and diesters of products of the addition of 1 to 30 moles of ethylene oxide to glycerol; glycerol monoesters and diesters and sorbitan monoesters and diesters of saturated and unsaturated fatty acids containing 6 to 22 carbon atoms and ethylene oxide adducts thereof; alkyl mono- and oligoglycosides containing 8 to 22 carbon atoms in the alkyl group and ethoxylated analogs thereof; products of the addition of 15 to 60 moles of ethylene oxide to castor oil and/or hydrogenated castor oil; polyol esters; products of the addition of 2 to 15 moles of ethylene oxide to castor oil and/or hydrogenated castor oil; partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols, alkyl glucosides and polyglucosides; trialkyl phosphates and mono-, di- and/or tri-PEG-alkyl phosphates; wool wax alcohols; polysiloxane/polyalkyl polyether copolymers; mixed esters of pentaerythritol, fatty acids, citric acid and fatty alcohol and/or mixed esters of fatty acids containing 6 to 22 carbon atoms, methyl glucose and polyols; hydrophobicized polyacrylates, polyalkylene glycols; betaines and esterquats.

6. Compositions as claimed in claim 5, characterized in that they contain alkyl oligoglucosides, betaines and/or polyglycerol poly-12-hydroxystearates as emulsifiers.

7. Compositions as claimed in claims 1 to 6, characterized in that they contain the dialkyl carbonates and the emulsifiers in quantities of 0.1 to 20% by weight, based on the compositions.

8. Compositions as claimed in claims 1 to 7, characterized in that they contain the dialkyl carbonates and the emulsifiers in a ratio by weight of 1:1 to 1:20.

9. Compositions as claimed in claims 1 to 8, characterized in that they additionally contain surfactants, oil components, superfatting agents, stabilizers, waxes, consistency factors, thickeners, cationic polymers, biogenic agents, antidandruff agents, film formers, preservatives, hydrotropes, solubilizers, UV filters, insect repellents, self-tanning agents, dyes and perfumes.

10. The use of the dialkyl carbonates claimed in claim 1 as a silicone oil substitute for the production of cosmetic and/or pharmaceutical compositions.

## Revendications

1. Préparations cosmétiques et/ou pharmaceutiques, contenant
(a) des carbonates de dialkyle de formule (I) 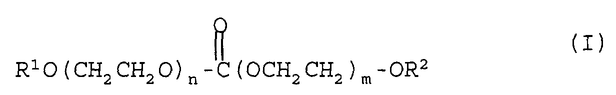 dans laquelle R¹ représente un radical alkyle et/ou alcényle linéaire ayant de 6 à 22 atomes de carbone, ou un radical qui dérive d'un polyol ayant de 2 à 15 atomes de carbone et au moins deux groupes hydroxy, R² représente R¹ ou le groupe méthyle, et n et m représentent, indépendamment l'un de l'autre, 0 ou des nombres de 1 à 100, et
(b) des émulsifiants.

2. Préparations selon la revendication 1,
caractérisées en ce qu'
elles contiennent des carbonates de dialkyle de formule (I) dans laquelle R¹ représente un radical alkyle linéaire ayant de 8 à 18 atomes de carbone, et R² représente R¹ ou le groupe méthyle.

3. Préparations selon la revendication 1,
caractérisées en ce qu'
elles contiennent des carbonates de dialkyle de formule (I) dans laquelle R¹ représente un radical alkyle linéaire ayant de 12 à 18 atomes de carbone, R² représente R¹ ou le groupe méthyle, et n et m représentent chacun des nombres de 1 à 10.

4. Préparations selon la revendication 1,
caractérisées en ce qu'
elles contiennent des carbonates de dialkyle de formule (I), dans laquelle R¹ représente un reste d'un polyol qui est choisi dans l'ensemble constitué par le glycérol, des alkylèneglycols, des mélanges industriels d'oligoglycérols, des composés de type méthylol, des (alkyl inférieur)glucosides, des alcools glucidiques, des glucides et des sucres aminés, et R² représente R¹, un radical alkyle linéaire ou ramifié ayant de 8 à 12 atomes de carbone, ou le groupe méthyle.

5. Préparations selon l'une quelconque des revendications 1 à 4,
caractérisées en ce qu'
elles contiennent des émulsifiants qui sont choisis dans l'ensemble constitué par les produits de fixation par addition de 2 à 30 moles d'oxyde d'éthylène et/ou 0 à 5 moles d'oxyde de propylène sur des alcools gras linéaires ayant de 8 à 22 atomes de carbone, sur des acides gras ayant de 12 à 22 atomes de carbone et sur des alkylphénols ayant de 8 à 15 atomes de carbone dans le groupe alkyle ; des mono- et diesters en C_{12/18} de produits de fixation par addition de 1 à 30 moles d'oxyde d'éthylène sur le glycérol ; des mono- et diesters de glycérol et des mono-et diesters de sorbitanne avec des acides gras saturés et des acides gras insaturés ayant de 6 à 22 atomes de carbone et leurs produits de fixation par addition d'oxyde d'éthylène ; des alkyl-mono- et -oligoglycosides ayant de 8 à 22 atomes de carbone dans le reste alkyle et leurs analogues éthoxylés ; des produits de fixation par addition de 15 à 60 moles d'oxyde d'éthylène sur l'huile de ricin et/ou l'huile de ricin hydrogénée ; des esters de polyols ; des produits de fixation par addition de 2 à 15 moles d'oxyde d'éthylène sur l'huile de ricin et/ou l'huile ricin hydrogénée ; des esters partiels à base d'acides gras en C_{6/22} linéaires, ramifiés, saturés ou insaturés, d'acide ricinoléique ainsi que d'acide 12-hydroxystéarique avec le glycérol, le polyglycérol, le pentaérythritol, le dipentaérythritol, des alcools glucidiques, des alkylglucosides ainsi que des polyglucosides ; des phosphates de trialkyle ainsi que des phosphates de mono-, di- et/ou tri-PEG-alkyle ; des alcolanums ; des copolymères polysiloxane/ polyalkyle/polyéther ; des esters mixtes de pentaérythritol, acides gras, acide citrique et alcools gras et/ou des esters mixtes d'acides gras ayant de 6 à 22 atomes de carbone, du méthylglucose et des polyols ; des polyacrylates rendus hydrophobes, des polyalkylèneglycols ; des bétaïnes et des esters quaternaires.

6. Préparations selon la revendication 5,
caractérisées en ce qu'
elles contiennent en tant qu'émulsifiants des alkyloligoglucosides, des bétaïnes et/ou des poly-12-hydroxystéarates de polyglycérol.

7. Préparations selon l'une quelconque des revendications 1 à 6,
caractérisées en ce qu'
elles contiennent les carbonates de dialkyle et les émulsifiants, chacun en proportions de 0,1 à 20 % en poids, par rapport aux préparations.

8. Préparations selon l'une quelconque des revendications 1 à 7,
caractérisées en ce qu'
elles contiennent les carbonates de dialkyle et les émulsifiants en un rapport pondéral de 1:1 à 1:20.

9. Préparations selon l'une quelconque des revendications 1 à 8,
caractérisées en ce qu'
elles contiennent en outre des tensioactifs, corps huileux, agents de surgraissage, stabilisants, cires, agents de consistance, épaississants, polymères cationiques, substances actives biogènes, agnts antipelliculaires, agents filmogènes, conservateurs, agents hydrotropes, solubilisants, filtres anti-UV, insectifuges, autobronzants, colorants et parfums.

10. Utilisation de carbonates de dialkyle selon la revendication 1, en tant que produit de remplacement de silicones, pour la fabrication de préparations cosmétiques et/ou pharmaceutiques.
